# EUROPEAN PATENT SPECIFICATION

(11) **EP 1 483 388 B1**
(45) Date of publication and mention of the grant of the patent: **05.11.2008**
(21) Application number: 03743821.5
(22) Date of filing: 27.02.2003
(51) Int. Cl.: C12N 15/53, C12N 9/02, C12P 13/08, C12P 13/04

(54) **PROCESS FOR THE PREPARATION OF L-AMINO ACIDS USING STRAINS OF THE ENTEROBACTERIACEAE FAMILY**
VERFAHREN ZUR HERSTELLUNG VON L-AMINOSÄUREN UNTER VERWENDUNG VON STÄMMEN DER FAMILIE ENTEROBACTERIACEAE
PROCEDE DE PREPARATION D'ACIDES L-AMINES AU MOYEN DE SOUCHES DE LA FAMILLE ENTEROBACTERIACEAE

(30) Priority: 13.03.2002 DE 10210960; 21.03.2002 US 365771 P
(43) Date of publication of application: 08.12.2004
(60) Divisional application: 08163518.7
(73) Proprietor: Evonik Degussa GmbH, 45128 Essen (DE)
(72) Inventor: RIEPING, Mechthild, 33619 Bielefeld (DE); HERMANN, Thomas, 33739 Bielefeld (DE); FARWICK, Mike, 45131 Essen (DE)
(86) International application number: PCT/EP2003/001996
(87) International publication number: WO 2003/076629

(56) References cited:
- EP-A- 0 643 135
- EP-A- 0 994 190
- WO-A-00/61723
- WO-A-01/16346
- WO-A-99/53035
- DE-A- 3 823 451
- US-A- 4 278 765
- HIDALGO E ET AL.: "Molecular cloning and DNA sequencing of the Escherichia coli K-12 ald gene encoding aldehyde dehydrogenase" JOURNAL OF BACTERIOLOGY, vol. 173, no. 19, October 1991 (1991-10), pages 6118-6123, XP008015902 ISSN: 0021-9193 cited in the application
- MICHAL G: "Biochemical pathways: an atlas of biochemistry and molecular biology" 1999, , JOHN WILEY & SONS INC. AND SPEKTRUM AKADEMISCHER VERLAG , NEW YORK - HEIDELBERG XP002242199 ISBN: 0-471-33130-9 figure 3.8-2 figures 4.2-1, 4.5-1 and 4.5-2
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551

## Description

### Field of the Invention

This invention relates to a process for the preparation of L-threonine, using strains of the Enterobacteriaceae family in which the aldA gene and optionally one or more of the genes selected from the group comprising aldB, aldH and betB is/are overexpressed.

### Prior Art

L-amino acids, in particular L-threonine, find application in human medicine and in the pharmaceuticals industry, in the foodstuffs industry and quite particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to technical fermentation measures, such as, for example, stirring and supply of oxygen, or to the composition of the nutrient media, such as, for example, the sugar concentration during fermentation, or to the reworking into product form, for example by ion-exchange chromatography, or to the intrinsic output properties of the microorganism itself.

Methods of mutagenesis, selection and mutant selection are used to improve the output properties of these microorganisms. Strains that are resistant to antimetabolites, such as, for example, the threonine analogue α-amino-β-hydroxyvaleric acid (AHV), or that are auxotrophic in respect of metabolites of regulatory importance and produce L-amino acid, such as, for example, L-threonine, are obtained in this manner.

Methods of recombinant DNA technology have also been employed for some years for strain improvement of strains of the Enterobacteriaceae family that produce L-amino acids, by amplifying individual amino-acid-biosynthesis genes and investigating the effect on production.

### Object of the Invention

The inventors had the object of providing new measures for improved preparation of L-threonine.

### Summary of the Invention

The invention provides a process for the preparation of L-threonine, using microorganisms of the Enterobacteriaceae family which, in particular, already produce L-threonine and in which the aldA gene and optionally one or more of the genes aldB, aldH and betB is/are overexpressed.

### Detailed Description of the Invention

The term "enhancement" in this connection describes the increase in the intracellular activity of one or more enzymes or proteins in a microorganism that are coded by the corresponding DNA, for example by increasing the number of copies of the gene or genes, using a strong promoter or a gene or allele that codes for a corresponding enzyme or protein with a high activity, and optionally combining these measures.

By the measures of enhancement, in particular over-expression, the activity or concentration of the corresponding protein is generally increased by at least 10 %, 25 %, 50 %, 75 %, 100 %, 150 %, 200 %, 300 %, 400 % or 500%, up to a maximum of 1000% or 2000%, relative to that of the wild-type protein or the activity or concentration of the protein in the initial microorganism.

The process is characterized in that the following steps are carried out:
a) fermentation of the microorganisms of the Enterobacteriaceae family which produce L-threonine and in which the alda gene and optionally one or more of the genes selected from the group comprising aldB, aldH and betB is/are over-expressed,
b) concentration of L-threonine in the medium or in the cells of the microorganisms, and
c) isolation of the desired L-amino acid, whereby constituents of the fermentation broth and/or the biomass in its entirety or in portions (> 0 to 100%) thereof optionally remain in the product.

The microorganismes that the present invention provides can produce L-threonine from glucose, sucrose, lactose, fructose, maltose, molasses, optionally starch, optionally cellulose or from glycerol and ethanol. They are representatives of the Enterobacteriaceae family selected from the genera Escherichia, Erwinia, Providencia and Serratia. The genera Escherichia and Serratia are preferred. Of the genus Escherichia the species Escherichia coli, and of the genus Serratia the species Serratia marcescens, are to be mentioned in particular.

Suitable strains, which produce L-threonine in particular, of the genus Escherichia, in particular of the species Escherichia coli, are, for example:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132.

Suitable L-threonine-producing strains of the genus Serratia, in particular of the species Serratia marcescens, are, for example:
Serratia marcescens HNr21
Serratia marcescens TLr156
Serratia marcescens T2000.

Strains from the Enterobacteriaceae family that produce L-threonine preferably have, inter alia, one or more genetic or phenotypic features selected from the group comprising: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues such as, for example, valine hydroxamate, resistance to purine analogues such as, for example, 6-dimethylaminopurine, a need for L-methionine, optionally a partial and compensable need for L-isoleucine, a need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally a capacity for sucrose utilization, enhancement of the threonine operon, enhancement of homoserine dehydrogenase I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic-acid formation.

It has been found that microorganisms of the Enterobacteriaceae family produce L-threonine in improved manner after over-expression, of the alda gene and optionally one or more of the genes selected from the group comprising aldB, aldH and betB.

The nucleotide sequences of the genes of Escherichia coli belong to the prior art (see the following text references) and can also be found in the genome sequence of Escherichia , coli published by Blattner et al. (Science 277: 1453-1462 (1997)).

The genes aldA, aldB, aldH and betB are described by, inter alia, the following data:

| | |
|---|---|
| aldA gene: Description: | Aldehyde dehydrogenase, NAD-dependent |
| EC No.: | 1.2.1.22 |
| Reference: | Hidalgo et al.; Journal of Bacteriology 173(19): 6118-6123 (1991) Limon et al.; International Microbiology 2(1): 33-38 (1999) |
| Accession No.: | AE000239 |
| Alternative gene name: | ald |
| aldB gene: | |
| Description: | Aldehyde dehydrogenase B, lactaldehyde dehydrogenase, breakdown of small molecules |
| EC No.: | (carbon compounds) 1.2.1.22 |
| Reference: | Xu and Johnson; Journal of Bacteriology 177(11): 3166-3175 (1995) |
| Accession No.: | AE000436 |
| aldH Gene: | |
| Description: | Aldehyde dehydrogenase, NADP-dependent |
| EC No.: | 1.2.1.3 |
| Reference: | Heim and Strehler; Gene 99(1): 15-23 (1991) |
| Accession No.: | AE000228 |
| betB gene: | |
| Description: | Betaine aldehyde dehydrogenase, NAD-dependent |
| EC No.: | 1.2.1.8 |
| Reference: Accession No.: | Lamark et al.; Molecular Microbiology 5(5): 1049-1064 (1991) Falkenberg and Strom; Biochimica et Biophysica Acta 1034(3): 253-259 (1990) AE000138 |

The nucleic-acid sequences can be found in the databases of the National Center for Biotechnology Information (NCBI) of the National Library of Medicine (Bethesda, MD, USA), in the Nucleotide Sequence Database of the European Molecular Biologies Laboratories (EMBL, Heidelberg, Germany or Cambridge, UK) or in the DNA Database of Japan (DDBJ, Mishima, Japan).

The genes described in the specified text references can be used in accordance with the invention. Alleles of the genes that arise from the degeneracy of the genetic code or as a result of functionally neutral sense mutations can furthermore be used. The use of endogenous genes is preferred.

"Endogenous genes" or "endogenous nucleotide sequences" are to be understood as meaning the genes or alleles or nucleotide sequences that are present in the population of a species.

To achieve an enhancement, the expression of the genes or the catalytic properties of the proteins can, for example, be enhanced. The two measures can optionally be combined.

To achieve an over-expression, the number of copies of the corresponding genes can be increased, or the promoter and regulation region or the ribosome-binding site upstream of the structural gene can be mutated. Expression cassettes that are incorporated upstream of the structural gene act in the same way. By means of inducible promoters it is additionally possible to intensify the expression in the course of fermentative production of L-threonine. The expression is likewise improved by measures to prolong the life of the mRNA. Furthermore, the enzyme activity is also increased by preventing the degradation of the enzyme protein. The genes or gene constructs may either be present in plasmids with a varying number of copies or may be integrated and amplified in the chromosome. Alternatively, an over-expression of the genes in question can furthermore be achieved by changing the composition of the media and the culture procedure.

A person skilled in the art can find instructions on this, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton et al. (Journal of Bacteriology 171: 4617-4622 (1989)), in Jensen and Hammer (Biotechnology and Bioengineering 58: 191-195 (1998)) and in known textbooks on genetics and molecular biology.

Plasmid vectors that can replicate in Enterobacteriaceae, such as, for example, cloning vectors derived from pACYC184 (Bartolomé et al.; Gene 102: 75-78 (1991)), pTrc99A (Amann et al.; Gene 69: 301-315 (1988)) or pSC101 derivatives (Vocke and Bastia, Proceedings of the National Academy of Sciences USA 80(21): 6557-6561 (1983)) can be used. A strain transformed with a plasmid vector, where the plasmid vector carries at least one or more of the genes selected from the group comprising aldA, aldB, aldH and betB, or nucleotide sequences that code for them, can be employed in a process according to the invention.

It is also possible to transfer mutations that affect the expression of the particular genes into various strains by sequence exchange (Hamilton et al.; (Journal of Bacteriology 171: 4617-4622 (1989)), conjugation or transduction.

It may furthermore be advantageous for the production of L-threonine with strains of the Enterobacteriaceae family to enhance one or more enzymes of the known threonine-biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the overexpression of the alda gene and optionally of one or more of the genes selected from the group comprising aldB, aldH and betB. The use of endogenous genes is generally preferred.

Thus, for example, at the same time one or more of the genes selected from the group comprising
- the thrABC operon which codes for aspartate kinase, homoserine dehydrogenase, homoserine kinase and threonine synthase (US-A-4,278,765),
- the pyc gene which codes for pyruvate carboxylase (DE-A-19 831 609),
- the pps gene which codes for phosphoenol pyruvate synthase (Molecular and General Genetics 231(2): 332-336 (1992)),
- the ppc gene which codes for phosphoenol pyruvate carboxylase (Gene 31: 279-283 (1984)),
- the pntA and pntB genes which code for transhydrogenase (European Journal of Biochemistry 158: 647-653 (1986)),
- the rhtB gene which imparts homoserine resistance (EP-A-0 994 190),
- the mqo gene which codes for malate:quinone oxidoreductase (DE 100 348 33.5),
- the rhtC gene which imparts threonine resistance (EP-A-1 013 765),
- the thrE gene of Corynebacterium glutamicum which codes for the threonine-export protein (DE 100 264 94.8),
- the gdhA gene which codes for glutamate dehydrogenase (Nucleic Acids Research 11: 5257-5266 (1983); Gene 23: 199-209 (1983)),
- the hns gene which codes for the DNA-binding protein HLP-II (Molecular and General Genetics 212: 199-202 (1988)),
- the pgm gene which codes for phosphoglucomutase (Journal of Bacteriology 176: 5847-5851 (1994)),
- the fba gene which codes for fructose biphosphate aldolase (Biochemical Journal 257: 529-534 (1989)),
- the ptsH gene of the ptsHIcrr operon which codes for the phosphohistidine protein hexose phosphotransferase of the phosphotransferase system PTS (Journal of Biological Chemistry 262: 16241-16253 (1987)),
- the ptsI gene of the ptsHIcrr operon which codes for enzyme I of the phosphotransferase system PTS (Journal of Biological Chemistry 262: 16241-16253 (1987)),
- the crr gene of the ptsHIcrr operon which codes for the glucose-specific IIA component of the phosphotransferase system PTS (Journal of Biological Chemistry 262: 16241-16253 (1987)),
- the ptsG gene which codes for the glucose-specific IIBC component (Journal of Biological Chemistry 261: 16398-16403 (1986)),
- the lrp gene which codes for the regulator of the leucine regulon (Journal of Biological Chemistry 266: 10768-10774 (1991)),
- the csrA gene which codes for the global regulator Csr (Journal of Bacteriology 175: 4744-4755 (1993)),
- the fadR gene which codes for the regulator of the fad regulon (Nucleic Acids Research 16: 7995-8009 (1988)),
- the iclR gene which codes for the regulator of central intermediary metabolism (Journal of Bacteriology 172: 2642-2649 (1990)),
- the mopB gene which codes for the 10 Kd chaperone (Journal of Biological Chemistry 261: 12414-12419 (1986)) and is also known by the name 'groES',
- the ahpC gene of the ahpCF operon which codes for the small subunit of alkyl hydroperoxide reductase (Proceedings of the National Academy of Sciences USA 92: 7617-7621 (1995)),
- the ahpF gene of the ahpCF operon which codes for the large subunit of alkyl hydroperoxide reductase (Proceedings of the National Academy of Sciences USA 92: 7617-7621 (1995)),
- the cysK gene which codes for cysteine synthase A (Journal of Bacteriology 170: 3150-3157 (1988)),
- the cysB gene which codes for the regulator of the cys regulon (Journal of Biological Chemistry 262: 5999-6005 (1987)),
- the cysJ gene of the cysJIH operon which codes for the flavoprotein of NADPH sulfite reductase (Journal of Biological Chemistry 264: 15796-15808 (1989), Journal of Biological Chemistry 264: 15726-15737 (1989)),
- the cysI gene of the cysJIH operon which codes for the haemoprotein of NADPH sulfite reductase (Journal of Biological Chemistry 264: 15796-15808 (1989), Journal of Biological Chemistry 264: 15726-15737 (1989)),
- the cysH gene of the cysJIH operon which codes for adenylyl sulfate reductase (Journal of Biological Chemistry 264: 15796-15808 (1989), Journal of Biological Chemistry 264: 15726-15737 (1989)),
- the phoB gene of the phoBR operon which codes for the positive regulator PhoB of the pho regulon (Journal of Molecular Chemistry 190 (1): 37-44 (1986)),
- the phoR gene of the phoBR operon which codes for the sensor protein of the pho regulon (Journal of Molecular Biology 192 (3): 549-556 (1986)),
- the phoE gene which codes for protein E of the outer cell membrane (Journal of Molecular Biology 163 (4): 513-532 (1983)),
- the pykF gene which codes for pyruvate kinase I which is stimulated by fructose (Journal of Bacteriology 177 (19): 5719-5722 (1995)),
- the pkfB gene which codes for 6-phosphofructokinase II (Gene 28 (3): 337-342 (1984)),
- the malE gene which codes for the periplasmic binding protein of maltose transport (Journal of Biological Chemistry 259 (16): 10606-10613 (1984)),
- the rseA gene of the rseABC operon which codes for a membrane protein with anti-sigmaE activity (Molecular Microbiology 24 (2): 355-371 (1997)),
- the rseC gene of the rseABC operon which codes for a global regulator of the sigmaE factor (Molecular Microbiology 24 (2): 355-371 (1997)),
- the sodA gene which codes for superoxide dismutase (Journal of Bacteriology 155 (3): 1078-1087 (1983)),
- the sucA gene of the sucABCD operon which codes for the decarboxylase subunit of 2-ketoglutarate dehydrogenase (European Journal of Biochemistry 141 (2): 351-359 (1984)),
- the sucB gene of the sucABCD operon which codes for the dihydrolipoyl transsuccinase E2 subunit of 2-ketoglutarate dehydrogenase (European Journal of Biochemistry 141 (2): 361-374 (1984)),
- the sucC gene of the sucABC operon which codes for the β-subunit of succinyl-CoA synthetase (Biochemistry 24 (22): 6245-6252 (1985)) and
- the sucD gene of the sucABCD operon which codes for the α-subunit of succinyl-CoA synthetase (Biochemistry 24 (22): 6245-6252 (1985))
can be over-expressed.

It may furthermore be advantageous for the production of L-threonine in addition to the alda gene and optionally of one or more of the genes selected from the group comprising aldB, aldH and betB, for one or more genes selected from the group comprising
- the tdh gene which codes for threonine dehydrogenase (Journal of Bacteriology 169: 4716-4721 (1987)),
- the mdh gene which codes for malate dehydrogenase (E.C. 1.1.1.37) (Archives in Microbiology 149: 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology Information (NCBI, Bethesda, MD, USA)),
- the pckA gene which codes for the enzyme phosphoenol pyruvate carboxykinase (Journal of Bacteriology 172: 7151-7156 (1990)),
- the poxB gene which codes for pyruvate oxidase (Nucleic Acids Research 14 (13): 5449-5460 (1986)),
- the aceA gene which codes for the enzyme isocitrate lyase (Journal of Bacteriology 170: 4528-4536 (1988)),
- the dgsA gene which codes for the DgsA regulator of the phosphotransferase system (Bioscience, Biotechnology and Biochemistry 59: 256-261 (1995)) and is also known by the name 'mlc gene',
- the fruR gene which codes for the fructose repressor (Molecular and General Genetics 226: 332-336 (1991)) and is also known by the name 'cra gene',
- the rpoS gene which codes for the sigma³⁸ factor (WO 01/05939) and is also known by the name 'katF gene',
- the aspA gene which codes for aspartate ammonium lyase (aspartase) (Nucleic Acids Research 13(6): 2063-2074 (1985)) and
- the aceB gene which codes for malate synthase A (Nucleic Acids Research 16(19): 9342 (1988))
to be eliminated.

The term "attenuation" in this connection describes the reduction or elimination of the intracellular activity of one or more enzymes (proteins) in a microorganism that are coded by the corresponding DNA, for example by using a weak promoter or a gene or allele that codes for a corresponding enzyme with a low activity or inactivates the corresponding enzyme (protein) or gene, and optionally combining these measures.

By the measures of attenuation, the activity or concentration of the corresponding protein is generally lowered to 0 to 75%, 0 to 50%, 0 to 25%, 0 to 10% or 0 to 5% of the activity or concentration of the wild-type protein, or of the activity or concentration of the protein in the initial microorganism.

It may furthermore be advantageous for the production of L-threonine in addition to the of the alda gene and optionally of one or more of the genes selected from the group comprising aldB, aldH and betB, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced in accordance with the invention can be cultured in the batch process (batch cultivation), in the fed-batch process or in the repeated-fed-batch process. A summary of known cultivation methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must satisfy the requirements of the particular strains in suitable manner. Descriptions of culture media for various microorganisms are contained in the handbook "Manual of Methods for General Bacteriology" of the American Society for Bacteriology (Washington D.C., USA, 1981).

Sugars and carbohydrates, such as, for example, glucose, sucrose, lactose, fructose, maltose, molasses, starch and optionally cellulose, oils and fats, such as, for example, soybean oil, sunflower oil, peanut oil and coconut oil, fatty acids, such as, for example, palmitic acid, stearic acid and linoleic acid, alcohols, such as, for example, glycerol and ethanol, and organic acids, such as, for example, acetic acid, can be used as the source of carbon. These substances can be used individually or as a mixture.

Organic nitrogenous compounds, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogenphosphate or dipotassium hydrogenphosphate or the corresponding sodium-containing salts can be used as the source of phosphorus. The culture medium must furthermore contain salts of metals, such as, for example, magnesium sulfate or iron sulfate, which are necessary for growth. Finally, essential growth substances, such as amino acids and vitamins, can be employed in addition to the above-mentioned substances. Suitable precursors can moreover be added to the culture medium. The named feed substances can be added to the culture in the form of a single charge or can be fed in during the cultivation in suitable manner.

The fermentation is generally carried out at a pH of 5.5 to 9.0, in particular 6.0 to 8.0. Basic compounds, such as sodium hydroxide; potassium hydroxide, ammonia or aqueous ammonia, or acidic compounds, such as phosphoric acid or sulfuric acid, can be employed in suitable manner to control the pH of the culture. Anti-foaming agents, such as, for example, fatty-acid polyglycol esters, can be employed to control the evolution of foam. Suitable substances having a selective action, for example antibiotics, can be added to the medium to maintain the stability of plasmids. To maintain aerobic conditions, oxygen or oxygenous gas mixtures, such as air for example, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be undertaken by anion-exchange chromatography with subsequent ninhydrin derivation, as described by Spackman et al. (Analytical Chemistry, 30: 1190-1206 (1958)), or it can be undertaken by reversed-phase HPLC, as described by Lindroth et al. (Analytical Chemistry 51: 1167-1174 (1979)).

The process according to the invention serves for the fermentative preparation of L-threonine.

The incubation-temperature in the preparation of strains and transformants is 37 °C.

### Example 1

### Preparation of L-threonine using the aldA gene

### 1a) Construction of the expression plasmid pTrc99AaldA

The aldA gene from E. coli K12 is amplified by applying the polymerase chain reaction (PCR) and also synthetic oligonucleotides. Proceeding from the nucleotide sequence of the aldA gene in E. coli K12 MG1655 (Accession Number AE000239, Blattner et al. (Science 277(5331): 1453-1474 (1997)), PCR primers are synthesized (MWG Biotech, Ebersberg, Germany). The sequences of the primers are modified in such a way that recognition sites for restriction enzymes arise. For the aldA1 primer the recognition sequence for XbaI is chosen, and for the aldA2 primer the recognition sequence for HindIII is chosen, these recognition sequences being marked by underlining in the nucleotide sequences represented below:
aldA1: 5'- CCGAAAACAAACATCTAGATCACAGGAG -3' (SEQ ID No. 1)
aldA2: 5'- CCTCCGAAGCTTTCACTCATTAAG -3' (SEQ ID No. 2)

The chromosomal E. coli K12 MG1655 DNA employed for the PCR is isolated, in accordance with the manufacturer's instructions, with "Qiagen Genomic-tips 100/G" (QIAGEN, Hilden, Germany). A DNA fragment having a size of about 1490 bp can be amplified with the specific primers under standard PCR conditions (Innis et al. (1990) PCR Protocols. A Guide to Methods and Applications, Academic Press) with *Taq*-DNA-Polymerase (QIAGEN, Hilden, Germany). The PCR product is ligated, according to the manufacturer's instructions, with the vector pCR®2.1-TOPO (TOPO TA Cloning Kit, Invitrogen, Groningen, Netherlands) and transformed into the E. coli strain TOP10F'. The selection of plasmid-carrying cells is undertaken on LB agar which is pricked with 50 µg/ml ampicillin. After the isolation of plasmid DNA, the vector is cleaved with the restriction enzymes EcoRI, BamHI and HindIII/XbaI and, after checking by fractionation in the 0.8% agarose gel, is designated by pCR2.1TOPOaldA.

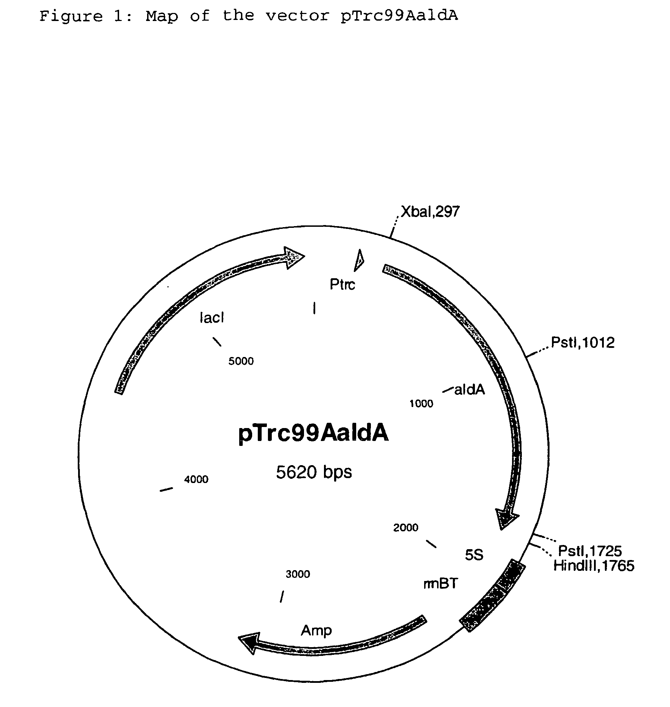

Subsequently the vector pCR2.1TOPOaldA is restricted with the restriction enzymes HindIII and XbaI, and the aldA fragment is isolated after fractionation in the 0.8% agarose gel with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligated with the isolated aldA fragment. The E. coli strain XL1-Blue MRF' (Stratagene, La Jolla, USA) is transformed with the ligation charge and selected in respect of plasmid-carrying cells on LB agar which is pricked with 50 µg/ml ampicillin. The successful cloning can be confirmed after the isolation of plasmid DNA by control cleavage with the enzymes HindIII/XbaI and PstI. The plasmid is designated as pTrc99AaldA (Figure 1).

### 1b) Preparation of L-threonine with the strain MG442/pTrc99AaldA

The E. coli strain MG442 which produces L-threonine is described in Patent US-A-4,278,765 and has been deposited in the Russian National Collection of Industrial Microorganisms (VKPM, Moscow, Russia) as CMIM B-1628.

The strain MG442 is transformed with the expression plasmid pTrc99AaldA described in Example 1a and with the vector pTrc99A and selected in respect of plasmid-carrying cells on LB agar with 50 µg/ml ampicillin. In this way the strains MG442/pTrc99AaldA and MG442/pTrc99A arise. Selected single colonies are subsequently multiplied further on minimal medium having the following composition: 3.5 g/l Na₂HPO₄.2H₂O, 1.5 g/l KH₂PO₄, 1 g/l NH₄Cl, 0.1 g/l MgSO₄.7H₂O, 2 g/l glucose, 20 g/l agar, 50 mg/l ampicillin. The formation of L-threonine is examined in batch cultures of 10 ml which are contained in 100 ml Erlenmeyer flasks. To this end, 10 ml of preculture medium having the following composition: 2 g/l yeast extract, 10 g/l (NH₄)₂SO₄, 1 g/l KH₂PO₄, 0.5 g/l MgSO₄.7H₂O, 15 g/l CaCO₃, 20 g/l glucose, 50 mg/l ampicillin, is inoculated and incubated for 16 hours at 37 °C and at 180 rpm in an ESR incubator manufactured by Kühner AG (Birsfelden, Switzerland). 250 µl at a time of this preculture are inoculated into 10 ml of production medium (25 g/l (NH₄)₂SO₄, 2 g/l KH₂PO₄, 1 g/l MgSO₄.7H₂0, 0.03 g/l FeSO₄.7H₂O, 0.018 g/l MnSO₄.1H₂O, 30 g/l CaCO₃, 20 g/l glucose, 50 mg/l ampicillin) and incubated for 48 hours at 37 °C. With a view to complete induction of the expression of the aldA gene, 100 mg/l isopropyl-β-D-thiogalactopyranoside (IPTG) are added in parallel charges. The formation of L-threonine by the parent strain MG442 is examined in the same way, but without addition of ampicillin to the medium. After the incubation, the optical density (OD) of the culture suspension is determined with an LP2W-Photometer manufactured by Dr.

Lange (Düsseldorf, Germany) at a measuring-wavelength of 660 nm.

Subsequently the concentration of L-threonine formed in the sterile-filtered culture supernatant is determined with an amino-acid analyzer manufactured by Eppendorf-BioTronik (Hamburg, Germany) by ion-exchange chromatography and post-column reaction with detection of ninhydrin.

The result of the experiment is presented in Table 1.

**Table 1**

| Strain | Admixtures | OD (660 nm) | L-threonine g/l |
|---|---|---|---|
| MG442 | - | 5.6 | 1.4 |
| MG442/pTrc99A | - | 3.8 | 1.3 |
| MG442/pTrc99AaldA | - | 5.6 | 1.8 |
| MG442/pTrc99AaldA | IPTG | 4.7 | 2.5 |

### Example 2

### Preparation of L-threonine using the aldB gene

### 2a) Construction of the expression plasmid pTrc99AaldB

The aldB gene from E. coli K12 is amplified by applying the polymerase chain reaction (PCR) and also synthetic oligonucleotides. Proceeding from the nucleotide sequence of the aldB gene in E. coli K12 MG1655 (Accession Number AE000436, Blattner et al. (Science 277(5331): 1453-1474 (1997)), PCR primers are synthesized (MWG Biotech, Ebersberg, Germany). The sequences of the primers are modified in such a way that recognition sites for restriction enzymes arise. For the aldB1 primer the recognition sequence for XbaI is chosen, and for the aldB2 primer the recognition sequence for HindIII is chosen, these recognition sequences being marked by underlining in the nucleotide sequences represented below:
aldB1: 5' - CAACTATCTCTAGAACCCTTGCCCG - 3' (SEQ ID No. 3)
aldB2: 5' - CCAATGCGACAAGCTTCTTATATC - 3' (SEQ ID No. 4)

The chromosomal E. coli K12 MG1655 DNA employed for the PCR is isolated, in accordance with the manufacturer's instructions, with "Qiagen Genomic-tips 100/G" (QIAGEN, Hilden, Germany). A DNA fragment having a size of about 1680 bp can be amplified with the specific primers under standard PCR conditions (Innis et al. (1990) PCR Protocols. A Guide to Methods and Applications, Academic Press) with *Taq*-DNA-Polymerase (QIAGEN, Hilden, Germany). The PCR product is ligated, according to the manufacturer's instructions, with the vector pCR®2.1-TOPO (TOPO TA Cloning Kit, Invitrogen, Groningen, Netherlands) and transformed into the E. coli strain TOP10F'. The selection of plasmid-carrying cells is undertaken on LB agar which is pricked with 50 µg/ml ampicillin. After the isolation of plasmid DNA, the vector is cleaved with the restriction enzymes XhoI and HindIII/XbaI and, after checking by fractionation in the 0.8% agarose gel, is designated by pCR2.1TOPOaldB.

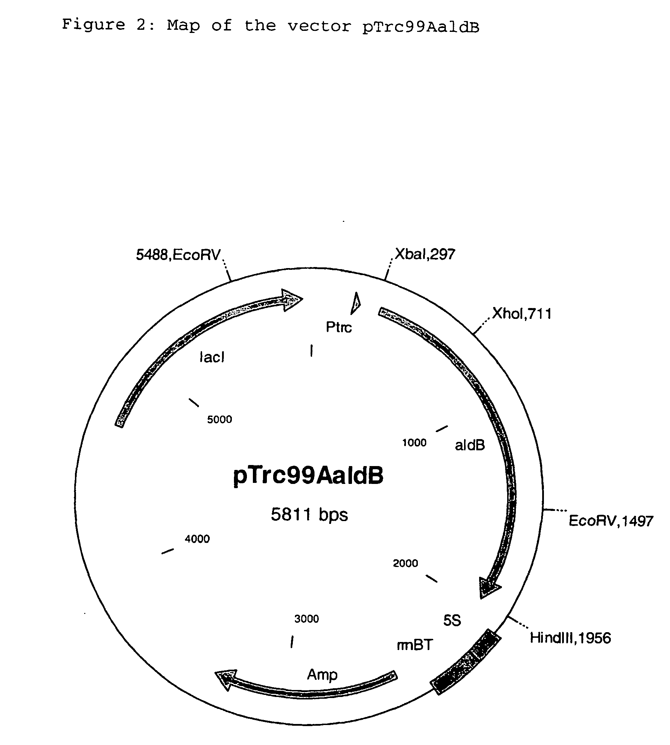

Subsequently the vector pCR2.1TOPoaldB is restricted with the restriction enzymes HindIII and XbaI, and the aldB fragment is isolated after fractionation in the 0.8% agarose gel with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligated with the isolated aldB fragment. The E. coli strain XL1-Blue MRF' (Stratagene, La Jolla, USA) is transformed with the ligation charge and selected in respect of plasmid-carrying cells on LB agar which is pricked with 50 µg/ml ampicillin. The successful cloning can be confirmed after the isolation of plasmid DNA by control cleavage with the enzymes HindIII/XbaI, EcoRV and XhoI. The plasmid is designated as pTrc99AaldB (Figure 2).

### 2b) Preparation of L-threonine with the strain MG442/pTrc99AaldB

The E. coli strain MG442 which produces L-threonine is described in Patent US-A-4,278,765 and has been deposited in the Russian National Collection of Industrial Microorganisms (VKPM, Moscow, Russia) as CMIM B-1628.

The strain MG442 is transformed with the expression plasmid pTrc99AaldB described in Example 2a and with the vector pTrc99A and selected in respect of plasmid-carrying cells on LB agar with 50 µg/ml ampicillin. In this way the strains MG442/pTrc99AaldB and MG442/pTrc99A arise. Selected single colonies are subsequently multiplied further on minimal medium having the following composition: 3.5 g/l Na₂HPO₄.2H₂O, 1.5 g/l KH₂PO₄, 1 g/l NH₄Cl, 0.1 g/l MgSO₄.7H₂O, 2 g/l glucose, 20 g/l agar, 50 mg/l ampicillin. The formation of L-threonine is examined in batch cultures of 10 ml which are contained in 100 ml Erlenmeyer flasks. To this end, 10 ml of preculture medium having the following composition: 2 g/l yeast extract, 10 g/l (NH₄)₂SO₄, 1 g/l KH₂PO₄, 0.5 g/l MgSO₄.7H₂0, 15 g/l CaCO₃, 20 g/l glucose, 50 mg/l ampicillin, is inoculated and incubated for 16 hours at 37 °C and at 180 rpm in an ESR incubator manufactured by Kühner AG (Birsfelden, Switzerland). 250 µl at a time of this preculture are inoculated into 10 ml of production medium (25 g/l (NH₄)₂SO₄, 2 g/l KH₂PO₄, 1 g/l MgSO₄.7H₂O, 0.03 g/l FeSO₄.7H₂O, 0.018 g/l MnSO₄.1H₂O, 30 g/l CaCO₃, 20 g/l glucose, 50 mg/l ampicillin) and incubated for 48 hours at 37 °C. With a view to complete induction of the expression of the aldB gene, 100 mg/l isopropyl-β-D-thiogalactopyranoside (IPTG) are added in parallel charges. The formation of L-threonine by the parent strain MG442 is examined in the same way, but without addition of ampicillin to the medium. After the incubation, the optical density (OD) of the culture suspension is determined with an LP2W-Photometer manufactured by Dr. Lange (Düsseldorf, Germany) at a measuring-wavelength of 660 nm.

Subsequently the concentration of L-threonine formed in the sterile-filtered culture supernatant is determined with an amino-acid analyzer manufactured by Eppendorf-BioTronik (Hamburg, Germany) by ion-exchange chromatography and post-column reaction with detection of ninhydrin.

The result of the experiment is presented in Table 2.

**Table 2**

| Strain | Admixtures | OD (660 nm) | L-threonine g/l |
|---|---|---|---|
| MG442 | - | 5.6 | 1.4 |
| MG442/pTrc99A | - | 3.8 | 1.3 |
| MG442/pTrc99AaldB | - | 4.6 | 2.0 |
| MG442/pTrc99AaldB | IPTG | 4.4 | 2.1 |

### Example 3

### Preparation of L-threonine using the aldH gene

### 3a) Construction of the expression plasmid pTrc99AaldH

The aldH gene from E. coli K12 is amplified by applying the polymerase chain reaction (PCR) and also synthetic oligonucleotides. Proceeding from the nucleotide sequence of the aldH gene in E. coli K12 MG1655 (Accession Number AE000228, Blattner et al. (Science 277(5331): 1453-1474 (1997)), PCR primers are synthesized (MWG Biotech, Ebersberg, Germany). The sequences of the primers are modified in such a way that recognition sites for restriction enzymes arise. For the aldH5 primer the recognition sequence for BamHI is chosen, and for the aldH3 primer the recognition sequence for SalI is chosen, these recognition sequences being marked by underlining in the nucleotide sequences represented below:
aldH3: 5'- AGCCGTCGACACCACGCAAACGTCGCAGG -3' (SEQ ID No. 5)
aldH5: 5'- ATCGGATCCTCTGCGCCCTCACGTTCACA -3' (SEQ ID No. 6)

The chromosomal E. coli K12 MG1655 DNA employed for the PCR is isolated, in accordance with the manufacturer's instructions, with "Qiagen Genomic-tips 100/G" (QIAGEN, Hilden, Germany). A DNA fragment having a size of about 1650 bp can be amplified with the specific primers under standard PCR conditions (Innis et al. (1990) PCR Protocols. A Guide to Methods and Applications, Academic Press) with *Taq*-DNA-Polymerase (QIAGEN, Hilden, Germany). The PCR product is ligated, according to the manufacturer's instructions, with the vector pCR-Blunt II-TOPO (Zero Blunt TOPO PCR Cloning Kit, Invitrogen, Groningen, Netherlands) and transformed into the E. coli strain TOP10. The selection of plasmid-carrying cells is undertaken on LB agar which is pricked with 50 µg/ml kanamycin. After the isolation of plasmid DNA, the vector is cleaved with the restriction enzymes BamHI and SalI and, after checking by fractionation in the 0.8% agarose gel, is designated by pCRBluntaldH.

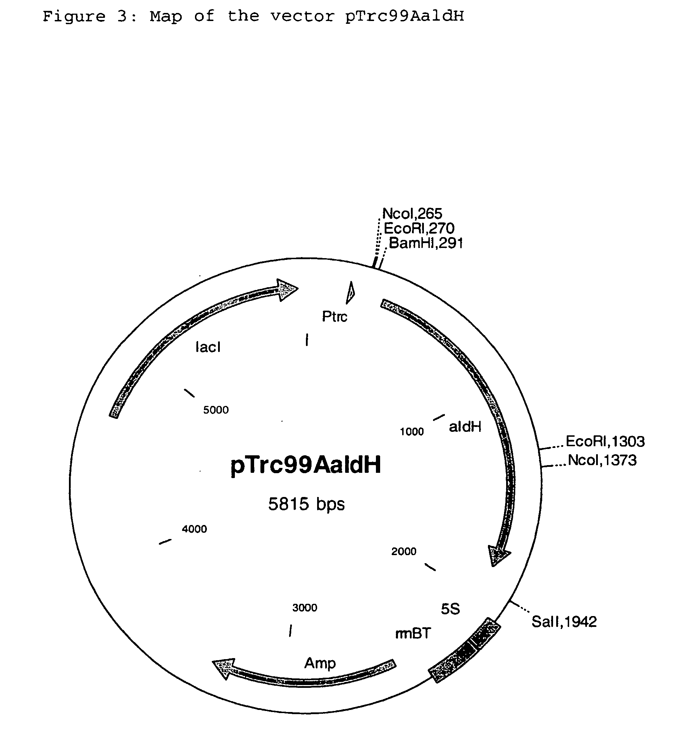

Subsequently the vector pCRBluntaldH is restricted with the restriction enzymes BamHI and SalI, and the aldH fragment is isolated after fractionation in the 0.8% agarose gel with the aid of the QIAquick Gel Extraction Kit (QIAGEN, Hilden, Germany). The vector pTrc99A (Amersham Biosciences, Freiburg, Germany) is cleaved with the enzymes BamHI and SalI, subsequently dephosphorylated with alkaline phosphatase according to the manufacturer's instructions (Amersham Biosciences, Freiburg, Germany) and ligated with the isolated aldH fragment. The E. coli strain XL1-Blue MRF' (Stratagene, La Jolla, USA) is transformed with the ligation charge and selected in respect of plasmid-carrying cells on LB agar which is pricked with 50 µg/ml ampicillin. The successful cloning can be confirmed after the isolation of plasmid DNA by control cleavage with the enzymes BamHI/SalI, EcoRI and NcoI. The plasmid is designated as pTrc99AaldH (Figure 3).

### 3b) Preparation of L-threonine with the strain MG442/pTrc99AaldH

The E. coli strain MG442 which produces L-threonine is described in Patent US-A-4,278,765 and has been deposited in the Russian National Collection of Industrial Microorganisms (VKPM, Moscow, Russia) as CMIM B-1628.

The strain MG442 is transformed with the expression plasmid pTrc99AaldH described in Example 3a and with the vector pTrc99A and selected in respect of plasmid-carrying cells on LB agar with 50 µg/ml ampicillin. In this way the strains MG442/pTrc99AaldH and MG442/pTrc99A arise. Selected single colonies are subsequently multiplied further on minimal medium having the following composition: 3.5 g/l Na₂HPO₄.2H₂O, 1.5 g/l KH₂PO₄, 1 g/l NH₄Cl, 0.1 g/l MgSO₄.7H₂O, 2 g/l glucose, 20 g/l agar, 50 mg/l ampicillin. The formation of L-threonine is examined in batch cultures of 10 ml which are contained in 100 ml Erlenmeyer flasks. To this end, 10 ml of preculture medium having the following composition: 2 g/l yeast extract, 10 g/l (NH₄)₂SO₄, 1 g/l KH₂PO₄, 0.5 g/l MgSO₄.7H₂O, 15 g/l CaCO₃, 20 g/l glucose, 50 mg/l ampicillin, is inoculated and incubated for 16 hours at 37 °C and at 180 rpm in an ESR incubator manufactured by Kühner AG (Birsfelden, Switzerland). 250 µl at a time of this preculture are inoculated into 10 ml of production medium (25 g/l (NH₄)₂SO₄, 2 g/l KH₂PO₄, 1 g/l MgSO₄.7H₂0, 0.03 g/l FeSO₄.7H₂O, 0.018 g/l MnSO₄.1H₂O, 30 g/l CaCO₃, 20 g/l glucose, 50 mg/l ampicillin) and incubated for 48 hours at 37 °C. The formation of L-threonine by the parent strain MG442 is examined in the same way, but without addition of ampicillin to the medium. After the incubation, the optical density (OD) of the culture suspension is determined with an LP2W-Photometer manufactured by Dr. Lange (Düsseldorf, Germany) at a measuring-wavelength of 660 nm.

Subsequently the concentration of L-threonine formed in the sterile-filtered culture supernatant is determined with an amino-acid analyzer manufactured by Eppendorf-BioTronik (Hamburg, Germany) by ion-exchange chromatography and post-column reaction with detection of ninhydrin.

The result of the experiment is presented in Table 3.

**Table 3**

| Strain | OD (660 nm) | L-threonine g/l |
|---|---|---|
| MG442 | 5.6 | 1.4 |
| MG442/pTrc99A | 3.8 | 1.3 |
| MG442/pTrc99AaldH | 5.6 | 3.3 |

### Example 4

### Preparation of L-threonine using the betB gene

### 4a) Construction of the expression plasmid pTrc99AbetB

The betB gene from E. coli K12 is amplified by applying the polymerase chain reaction (PCR) and also synthetic oligonucleotides. Proceeding from the nucleotide sequence of the betB gene in E. coli K12 MG1655 (Accession Number AE000138, Blattner et al. (Science 277: 1453-1474 (1997)), PCR primers are synthesized (MWG Biotech, Ebersberg, Germany). The sequences of the primers are modified in such a way that recognition sites for restriction enzymes arise. For the betB1 primer the recognition sequence for XbaI is chosen, and for the betB2 primer the recognition sequence for HindIII is chosen, these recognition sequences being marked by underlining in the nucleotide sequences represented below:
betB1: 5' - CAGCATCTAGACACCGATTAACCGAG - 3' (SEQ ID No. 7)
betB2: 5' - CAAATTGCAAATAAAGCTTCTGGTTAG - 3' (SEQ ID No. 8)

The chromosomal E. coli K12 MG1655 DNA employed for the PCR is isolated, in accordance with the manufacturer's instructions, with "Qiagen Genomic-tips 100/G" (QIAGEN, Hilden, Germany). A DNA fragment having a size of about 1530 bp can be amplified with the specific primers under standard PCR conditions (Innis et al. (1990) PCR Protocols. A Guide to Methods and Applications, Academic Press) with *Taq*-DNA-Polymerase (QIAGEN, Hilden, Germany). The PCR product is ligated, according to the manufacturer's instructions, with the vector pCR®2.1-TOPO (TOPO TA Cloning Kit, Invitrogen, Groningen, Netherlands) and transformed into the E. coli strain TOP10F'. The selection of plasmid-carrying cells is undertaken on LB agar which is pricked with 50 µg/ml ampicillin. After the isolation of plasmid DNA, the vector is cleaved with the restriction enzymes EcoRI and ScaI and, after checking by fractionation in the 0.8% agarose gel, is designated by pCR2.1TOPObetB.

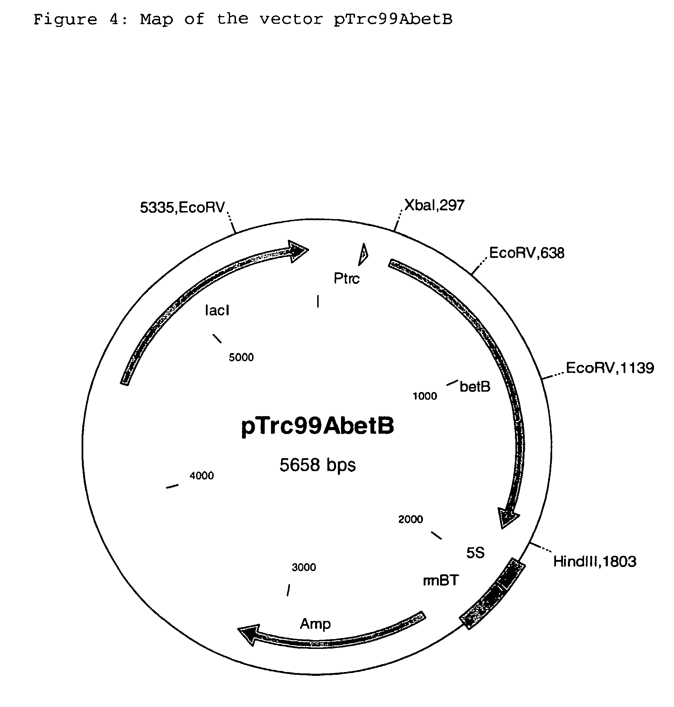

Subsequently the vector pCR2.iTOPObetB is restricted with the restriction enzymes HindIII and XbaI, and the betB fragment is isolated after fractionation in the 0.8% agarose gel with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligated with the isolated betB fragment. The E. coli strain XL1-Blue MRF' (Stratagene, La Jolla, USA) is transformed with the ligation charge and selected in respect of plasmid-carrying cells on LB agar which is pricked with 50 µg/ml ampicillin. The successful cloning can be confirmed after the isolation of plasmid DNA by control cleavage with the enzymes EcoRV, HindIII and XbaI. The plasmid is designated as pTrc99AbetB (Figure 4).

### 4b) Preparation of L-threonine with the strain MG442/pTrc99AbetB

The E. coli strain MG442 which produces L-threonine is described in Patent US-A-4,278,765 and has been deposited in the Russian National Collection of Industrial Microorganisms (VKPM, Moscow, Russia) as CMIM B-1628.

The strain MG442 is transformed with the expression plasmid pTrc99AbetB described in Example 4a and with the vector pTrc99A and selected in respect of plasmid-carrying cells on LB agar with 50 µg/ml ampicillin. In this way the strains MG442/pTrc99AbetB and MG442/pTrc99A arise. Selected single colonies are subsequently multiplied further on minimal medium having the following composition: 3.5 g/l Na₂HPO₄.2H₂O, 1.5 g/l KH₂PO₄, 1 g/l NH₄Cl, 0.1 g/l MgSO₄.7H₂O, 2 g/l glucose, 20 g/l agar, 50 mg/l ampicillin. The formation of L-threonine is examined in batch cultures of 10 ml which are contained in 100 ml Erlenmeyer flasks. To this end, 10 ml of preculture medium having the following composition: 2 g/l yeast extract, 10 g/l (NH₄)2SO₄, 1 g/l KH₂PO₄, 0.5 g/l MgSO₄.7H₂0, 15 g/l CaCO₃, 20 g/l glucose, 50 mg/l ampicillin, is inoculated and incubated for 16 hours at 37 °C and at 180 rpm in an ESR incubator manufactured by Kühner AG (Birsfelden, Switzerland). 250 µl at a time of this preculture are inoculated into 10 ml of production medium (25 g/l (NH₄)₂SO₄, 2 g/l KH₂PO₄, 1 g/l MgSO₄.7H₂0, 0.03 g/l FeSO₄.7H₂O, 0.018 g/l MnSO₄.1H₂O, 30 g/l CaCO₃, 20 g/l glucose, 50 mg/l ampicillin) and incubated for 48 hours at 37 °C. The formation of L-threonine by the parent strain MG442 is examined in the same way, but without addition of ampicillin to the medium. After the incubation, the optical density (OD) of the culture suspension is determined with an LP2W-Photometer manufactured by Dr. Lange (Düsseldorf, Germany) at a measuring-wavelength of 660 nm.

Subsequently the concentration of L-threonine formed in the sterile-filtered culture supernatant is determined with an amino-acid analyzer manufactured by Eppendorf-BioTronik (Hamburg, Germany) by ion-exchange chromatography and post-column reaction with detection of ninhydrin.

The result of the experiment is presented in Table 4.

**Table 4**

| Strain | OD (660 nm) | L-threonine g/l |
|---|---|---|
| MG442 | 5.6 | 1.4 |
| MG442/pTrc99A | 3.8 | 1.3 |
| MG442/pTrc99AbetB | 4.2 | 2.6 |

### Brief Description of the Figures:

Figure 1: Map of the vector pTrc99AaldA
Figure 2: Map of the vector pTrc99AaldB
Figure 3: Map of the vector pTrc99AaldH
Figure 4: Map of the vector pTrc99AbetB

Length data are to be interpreted as approximate data. The abbreviations and designations that are used have the following meanings:
- Amp: ampicillin-resistance gene
- lacI: gene for the repressor protein of the trc promoter
- Ptrc: trc promoter region, IPTG-inducible
- aldA: coding region of the aldA gene
- aldB: coding region of the aldB gene
- aldH: coding region of the aldH gene
- betB: coding region of the betB gene
- 5S: 5S rRNA region
- rrnBT: rRNA terminator region

The abbreviations for the restriction enzymes have the following meanings:
- BamHI: restriction endonuclease derived from *Bacillus amyloliquefaciens* H
- EcoRI: restriction endonuclease derived from *Escherichia coli* RY13
- EcoRV: restriction endonuclease derived from *Escherichia coli* B946
- HindIII: restriction endonuclease derived from *Haemophilus influenzae*
- NcoI: restriction endonuclease derived from *Rhodococcus* sp. ATCC® 19070
- PstI: restriction endonuclease derived from *Providencia stuartii*
- SalI: restriction endonuclease derived from *Streptomyces albus* G
- XbaI: restriction endonuclease derived from *Xanthomonas badrii*
- XhoI: restriction endonuclease derived from *Xanthomonas holcicola*

## Claims

1. A process for the preparation of L-threonine,
wherein the following steps are carried out:
a) fermentation of microorganisms of the Enterobacteriaceae family which produce the desired L-amino acid and in which the aldA gene encoding aldehyde dehydrogenase (NAD dependent) is over-expressed,
b) concentration of the desired L-amino acid in the medium or in the cells of the microorganisms, and
c) isolation of the desired L-amino acid, whereby constituents of the fermentation broth and/or the biomass in its entirety or portions (> 0 to 100%) thereof optionally remain in the product.

2. Process according to claim 1, wherein the polynucleotides of one or more genes selected from the group comprising aldB, aldH and betB is (are) over-expressed, whereby aldB encodes aldehyde dehydrogenase B, aldH encodes aldehyde dehydrogenase, NADP dependent and betB encodes betaine aldehyde dehydrogenase, NAD dependent.

3. Process according to claim 1, wherein for the preparation of L-threonine microorganisms of the Enterobacteriaceae family are fermented in which additionally at the same time one or more of the genes selected from the group comprising:
3.1 the thrABC operon which codes for aspartate kinase, homoserine dehydrogenase, homoserine kinase and threonine synthase,
3.2 the pyc gene which codes for pyruvate carboxylase,
3.3 the pps gene which codes for phosphoenolpyruvate synthase,
3.4 the ppc gene which codes for phosphoenolpyruvate carboxylase,
3.5 the pntA and pntB genes which code for transhydrogenase,
3.6 the Escherichia coli rhtB gene coding for a protein imparting homoserine resistance,
3.7 the mqo gene which codes for malate:quinone oxidoreductase,
3.8 the Escherichia coli rhtC gene coding for a protein imparting threonine resistance,
3.9 the thrE gene which codes for the threonine export protein,
3.10 the gdhA gene which codes for glutamate dehydrogenase,
3.11 the hns gene which codes for the DNA-binding protein HLP-II,
3.12 the pgm gene, which codes for phosphoglucoisomerase,
3.13 the fba gene which codes for fructose bisphosphate aldolase,
3.14 the ptsH gene which codes for the phosphohistidine protein hexose phosphotransferase,
3.15 the ptsI gene which codes for enzyme I of the phosphotransferase system,
3.16 the crr gene which codes for the glucose-specific IIA component,
3.17 the ptsG gene which codes for the glucose-specific IIBC component,
3.18 the lrp gene, which codes for the regulator of the leucine regulon,
3.19 the csrA gene, which codes for the global regulator Csr,
3.20 the fadR gene, which codes for the regulator FadR of fatty acid and acetate metabolism,
3.21 the iclR gene, which codes for the regulator IclR,
3.22 the mopB gene, which codes for the 10 kD chaperone,
3.23 the ahpC gene, which codes for the small subunit of alkyl hydroperoxide reductase,
3.24 the ahpF gene, which codes for the large subunit of alkyl hydroperoxide reductase,
3.25 the cysK gene, which codes for cysteine synthase A,
3.26 the cysB gene, which codes for the regulator of the cys regulon,
3.27 the cysJ gene, which codes for the flavoprotein of NADPH-sulfite reductase,
3.28 the cysI gene, which codes for the haemoprotein of NADPH-sulfite reductase,
3.29 the cysH gene, which codes for adenylyl sulfate reductase,
3.30 the phoB gene, which codes for the positive regulator PhoB of the pho regulon,
3.31 the phoR gene, which codes for the sensor protein of the pho regulon,
3.32 the phoE gene, which codes for protein E of the outer cell membrane,
3.33 the pykF gene, which codes for fructose-stimulated pyruvate kinase I,
3.34 the pfkB gene, which codes for 6-phosphofructokinase II,
3.35 the malE gene, which codes for the periplasmatic binding protein of maltose transport,
3.36 the rseA gene, which codes for a membrane protein which acts as a negative regulator on sigmaE activity,
3.37 the rseC gene, which codes for a global regulator of the sigmaE factor,
3.38 the sodA gene, which codes for superoxide dismutase,
3.39 the sucA gene, which codes for the decarboxylase subunit of 2-ketoglutarate dehydrogenase,
3.40 the sucB gene, which codes for the dihydrolipoyltranssuccinase E2 subunit of 2-ketoglutarate dehydrogenase,
3.41 the sucC gene, which codes for the β-subunit of succinyl-CoA synthetase and
3.42 the sucD gene, which codes for the α-subunit of succinyl-CoA synthetase
is or are over-expressed.

4. Process according to claim 1, wherein for the preparation of L-threonine microorganisms of the Enterobacteriaceae family are fermented in which additionally at the same time one or more of the genes selected from the group comprising:
4.1 the tdh gene which codes for threonine dehydrogenase,
4.2 the mdh gene which codes for malate dehydrogenase,
4.3 the gene product of the open reading frame (orf) yjfA of E. coli, when said microorganism is from the species E. coli,
4.4 the gene product of the open reading frame (orf) ytfP of E. coli, when said microorganism is from the species E. coli,
4.5 the pckA gene which codes for phosphoenolpyruvate carboxykinase,
4.6 the poxB gene which codes for pyruvate oxidase,
4.7 the aceA gene which codes for isocitrate lyase,
4.8 the dgsA gene which codes for the DgsA regulator of the phosphotransferase system,
4.9 the fruR gene which codes for the fructose repressor,
4.10 the rpoS gene which codes for the sigma³⁸ factor,
4.11 the aspA gene which codes for the aspartate ammonia-lyase (aspartase) and
4.12 the aceB gene which codes for malate synthase A
is or are eliminated.

5. Transformed microorganisms of the genus Escherichia, in which the aldA gene and one or more of the genes selected from the group comprising aldB, aldH and betB are present in over-expressed form, and which produce L-threonine.

6. Microorganisms according to claim 5, wherein the microorganisms originate from the species E. coli.

## Patentansprüche

1. Verfahren zur Herstellung von L-Threonin, bei dem man folgende Schritte durchführt:
a) Fermentation von die gewünschte L-Aminosäure produzierenden Mikroorganismen der Familie Enterobacteriaceae, in denen das für die Aldehyd-Dehydrogenase (NADP-abhängig) kodierende aldA-Gen überexprimiert ist,
b) Anreicherung der gewünschten L-Aminosäure im Medium oder in den Zellen der Mikroorganismen und
c) Isolierung der gewünschten L-Aminosäure, wodurch gegebenenfalls Bestandteile der Fermentationsbrühe und/oder die Biomasse in ihrer Gesamtheit oder Anteilen (> 0 bis 100%) davon im Produkt verbleiben.

2. Verfahren nach Anspruch 1, wobei die Polynukleotide eines oder mehrerer Gene, ausgewählt aus der Gruppe aldB, aldH und betB, überexprimiert wird (werden), wobei aldB für die Aldehyd-Dehydrogenase B, aldH für die Aldehyd-Dehydrogenase, NADP-abhängig, und betB für die Betainaldehyd-Dehydrogenase, NAD-abhängig, kodiert.

3. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
3.1 das für die Aspartatkinase, die Homoserin-Dehydrogenase, die Homoserinkinase und die Threoninsynthase kodierende thrABC-Operon,
3.2 das für die Pyruvat-Carboxylase kodierende pyc-Gen,
3.3 das für die Phosphoenolpyruvat-Synthase kodierende pps-Gen,
3.4 das für die Phosphoenolpyruvat-Carboxylase kodierende ppc-Gen,
3.5 die für die Transhydrogenase kodierenden Gene pntA und pntB,
3.6 das für ein Homoserinresistenz vermittelndes Protein kodierende Gen rhtB von Escherichia coli,
3.7 das für die Malat:Chinon Oxidoreduktase kodierende mqo-Gen,
3.8 das für ein Threoninresistenz vermittelndes Protein kodierende Gen rhtC von Escherichia coli,
3.9 das für das Threoninexportprotein kodierende thrE-Gen,
3.10 das für die Glutamat-Dehydrogenase kodierende gdhA-Gen,
3.11 das für das DNA-Bindeprotein HLP-II kodierende hns-Gen,
3.12 das für die Phosphoglukoisomerase kodierende pgm-Gen,
3.13 das für die Fructose-Bisphosphataldolase kodierende fba-Gen,
3.14 das für die Phosphohistidinproteinhexosephosphotransferase kodierende ptsH-Gen,
3.15 das für Enzym I des Phosphotransferase-Systems kodierende ptsI-Gen,
3.16 das für die glukosespezifische IIA-Komponente kodierende crr-Gen,
3.17 das für die glukosespezifische IIBC Komponente kodierende ptsG-Gen,
3.18 das für den Regulator des Leucin-Regulons kodierende lrp-Gen,
3.19 das für den globalen Regulator Csr kodierende csrA-Gen,
3.20 das für den Regulator FadR des Fettsäure- und Acetatstoffwechsels kodierende fadR-Gen.
3.21 das für den Regulator IclR kodierende iclR-Gen,
3.22 das für das für das Chaperon 10 kD kodierende mopB-Gen,
3.23 das für die kleine Untereinheit der Alkylhydroperoxidreduktase kodierende ahpC-Gen,
3.24 das für die große Untereinheit der Alkylhydroperoxidreduktase kodierende ahpF-Gen,
3.25 das für die Cystein-Synthase A kodierende cysK-Gen,
3.26 das für den Regulator des cys-Regulons kodierende cysB-Gen,
3.27 das für das Flavoprotein der NADPH-Sulfitreduktase kodierende cysJ-Gen,
3.28 das für das Hämoprotein der NADPH-Sulfitreduktase kodierende cysI-Gen,
3.29 das für die Adenylylsulfat-Reduktase kodierende cysH-Gen,
3.30 das für den positiven Regulator PhoB des pho Regulons kodierende phoB-Gen,
3.31 das für das Sensorprotein des pho Regulons kodierende phoR-Gen,
3.32 das für das Protein E der äußeren Zellmembran kodierende phoE-Gen,
3.33 das für die fructosestimulierte Pyruvatkinase I kodierende pykF-Gen,
3.34 das für die 6-Phosphofructokinase II kodierende pfkB-Gen,
3.35 das für das periplasmatische Bindungsprotein des Maltosetransports kodierende malE-Gen,
3.36 das für ein Membranprotein, das als negativer Regulator auf die sigmaE-Aktivität wirkt, kodierende rseA-Gen
3.37 das für einen globalen Regulator des sigmaE-Faktors kodierende rseC-Gen,
3.38 das für die Superoxid-Dismutase kodierende sodA-Gen,
3.39 das für die Decarboxylase-Untereinheit von 2-Ketoglutarat-Dehydrogenase kodierende sucA-Gen,
3.40 das für die Dihydrolipolytranssuccinase E2-Untereinheit von 2-Ketoglutarat-Dehydrogenase kodierende sucB-Gen,
3.41 das für die β-Untereinheit von Succinyl-CoA-Synthetase kodierende sucC-Gen,
3.42 das für die α-Untereinheit von Succinyl-CoA-Synthetase kodierende sucD-Gen,
überexprimiert.

4. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
4.1 das für die Threonin-Dehydrogenase kodierende tdh-Gen,
4.2 das für die Malat-Dehydrogenase kodierende mdh-Gen,
4.3 falls der genannte Mikroorganismus aus der Spezies E. coli ist, das Genprodukt des offenen Leserahmens (orf) yjfA von E. coli,
4.4 falls der genannte Mikroorganismus aus der Spezies E. coli ist, das Genprodukt des offenen Leserahmens (orf) ytfP von E. coli,
4.5 das für die Phosphoenolpyruvat-Carboxykinase kodierende pckA-Gen,
4.6 das für die Pyruvat-Oxidase kodierende poxB-Gen,
4.7 das für die Isocitrat-Lyase kodierende aceA-Gen,
4.8 das für den DgsA-Regulator des Phosphotransferase-Systems kodierende dgsA-Gen,
4.9 das für den Fructose-Repressor kodierende fruR-Gen,
4.10 das für den Faktor Sigma³⁸ kodierende rpoS-Gen,
4.11 das für die Aspartat-Ammoniak-Lyase (Aspartase) kodierende aspA-Gen und
4.12 das für die Malatsynthase A kodierende aceB-Gen
ausschaltet.

5. Transformierte Mikroorganismen der Gattung Echerichia, bei denen das aldA-Gen und eines oder mehrere der Gene, ausgewählt aus der Gruppe aldB, aldH und betB, in überexprimierter Form vorliegen und die L-Threonin produzieren.

6. Mikroorganismen nach Anspruch 5, bei denen die Mikroorganismen aus der Spezies E. coli sind.

## Revendications

1. Procédé de préparation de L-thréonine, selon lequel les étapes suivantes sont réalisées :
a) la fermentation de microorganismes de la famille des Enterobacteriaceae qui produisent l'acide L-aminé souhaité et dans lesquels le gène aldA qui code pour l'aldéhyde déshydrogénase (NAD-dépendante) est surexprimé,
b) la concentration de l'acide L-aminé souhaité dans le milieu ou dans les cellules des microorganismes, et
c) l'isolement de l'acide L-aminé souhaité, les constituants du bouillon de fermentation et/ou la biomasse en totalité ou en partie (> 0 à 100 %) restant éventuellement dans le produit.

2. Procédé selon la revendication 1, selon lequel les polynucléotides d'un ou de plusieurs gènes choisis dans le groupe comprenant aldB, aldH et betB sont surexprimés, aldB codant pour l'aldéhyde déshydrogénase B, aldH codant pour l'aldéhyde déshydrogénase NADP-dépendante et betB codant pour la bétaïne aldéhyde déshydrogénase NAD-dépendante.

3. Procédé selon la revendication 1, selon lequel, pour la préparation de L-thréonine, des microorganismes de la famille des Enterobacteriaceae sont fermentés, microorganismes dans lesquels, en outre, en même temps, un ou plusieurs des gènes choisis dans le groupe constitué par :
3.1 l'opéron thrABC qui code pour l'aspartate kinase, l'homosérine déshydrogénase, l'homosérine kinase et la thréonine synthase,
3.2 le gène pyc qui code pour la pyruvate carboxylase,
3.3 le gène pps qui code pour la phosphoénolpyruvate synthase,
3.4 le gène ppc qui code pour la phosphoénolpyruvate carboxylase,
3.5 les gènes pntA et pntB qui codent pour la transhydrogénase,
3.6 le gène rhtB d'Escherichia coli codant pour une protéine qui confère une résistance à l'homosérine,
3.7 le gène mqo qui code pour la malate:quinone oxydoréductase,
3.8 le gène rhtC d'Escherichia coli codant pour une protéine qui confère une résistance à la thréonine,
3.9 le gène thrE qui code pour la protéine d'exportation de la thréonine,
3.10 le gène gdhA qui code pour la glutamate déshydrogénase,
3.11 le gène hns qui code pour la protéine HLP-II se liant à l'ADN,
3.12 le gène pgm qui code pour la phosphoglucoisomérase,
3.13 le gène fba qui code pour la fructose bisphosphate aldolase,
3.14 le gène ptsH qui code pour la phosphohistidine protéine hexose phosphotransférase,
3.15 le gène ptsI qui code pour l'enzyme I du système phosphotransférase,
3.16 le gène crr qui code pour le composant IIA spécifique du glucose,
3.17 le gène ptsG qui code pour le composant IIBC spécifique du glucose,
3.18 le gène lrp qui code pour le régulateur du régulon de la leucine,
3.19 le gène csrA qui code pour le régulateur global Csr,
3.20 le gène fadR qui code pour le régulateur FadR du métabolisme des acides gras et de l'acétate,
3.21 le gène iclR qui code pour le régulateur IclR,
3.22 le gène mopB qui code pour la protéine chaperonne de 10 kD,
3.23 le gène ahpC qui code pour la petite sous-unité de l'alkyl hydroperoxyde réductase,
3.24 le gène ahpF qui code pour la grande sous-unité de l'alkyl hydroperoxyde réductase,
3.25 le gène cysK qui code pour la cystéine synthase A,
3.26 le gène cysB qui code pour le régulateur du régulon cys,
3.27 le gène cysJ qui code pour la flavoprotéine de la NADPH sulfite réductase,
3.28 le gène cysI qui code pour l'hémoprotéine de la NADPH sulfite réductase,
3.29 le gène cysH qui code pour l'adénylylsulfate réductase,
3.30 le gène phoB qui code pour le régulateur positif PhoB du régulon pho,
3.31 le gène phoR qui code pour la protéine capteur du régulon pho,
3.32 le gène phoE qui code pour la protéine E de la membrane cellulaire externe,
3.33 le gène pykF qui code pour la pyruvate kinase I stimulée par le fructose,
3.34 le gène pfkB qui code pour la 6-phosphofructokinase II,
3.35 le gène malE qui code pour la protéine de liaison périplasmique du transport du maltose,
3.36 le gène rseA qui code pour une protéine membranaire qui joue le rôle de régulateur négatif sur l'activité de sigmaE,
3.37 le gène rseC qui code pour un régulateur global du facteur sigmaE,
3.38 le gène sodA qui code pour la superoxyde dismutase,
3.39 le gène sucA qui code pour la sous-unité décarboxylase de la 2-cétoglutarate déshydrogénase,
3.40 le gène sucB qui code pour la sous-unité dihydrolipoyltranssuccinase E2 de la 2-cétoglutarate déshydrogénase,
3.41 le gène sucC qui code pour la sous-unité β de la succinyl-CoA synthétase, et
3.42 le gène sucD qui code pour la sous-unité α de la succinyl-CoA synthétase
est ou sont surexprimés.

4. Procédé selon la revendication 1, selon lequel, pour la préparation de L-thréonine, des microorganismes de la famille des Enterobacteriaceae sont fermentés, microorganismes dans lesquels, en outre, en même temps, un ou plusieurs des gènes choisis dans le groupe constitué par :
4.1 le gène tdh qui code pour la thréonine déshydrogénase,
4.2 le gène mdh qui code pour la malate déshydrogénase,
4.3 le produit génique du cadre ouvert de lecture (orf) yjfA d'E. coli, lorsque ledit microorganisme est de l'espèce E. coli,
4.4 le produit génique du cadre ouvert de lecture (orf) ytfP d'E. coli, lorsque ledit microorganisme est de l'espèce E. coli,
4.5 le gène pckA qui code pour la phosphoénolpyruvate carboxykinase,
4.6 le gène poxB qui code pour la pyruvate oxydase,
4.7 le gène aceA qui code pour l'isocitrate lyase,
4.8 le gène dgsA qui code pour le régulateur DgsA du système phosphotransférase,
4.9 le gène fruR qui code pour le répresseur du fructose, et
4.10 le gène rpoS qui code pour le facteur sigma³⁸,
4.11 le gène aspA qui code pour l'aspartate ammonia-lyase (aspartase), et
4.12 le gène aceB qui code pour la malate synthase A
est ou sont éliminés.

5. Microorganismes transformés du genre Escherichia, dans lesquels le gène aldA et un ou plusieurs des gènes choisis dans le groupe comprenant aldB, aldH et betB sont présents sous forme surexprimée, et qui produisent de la L-thréonine.

6. Microorganismes selon la revendication 5, les microorganismes provenant de l'espèce E. coli.


### Sequence Listing

<110> Degussa AG
<120> Process for the preparation of L-amino acids using strains of the Enterobacteriaceae family
<130> 020125 BT
<160> 8
<170> PatentIn version 3.1
<210> 1
   <211> 28
   <212> DNA
   <213> synthetic sequence
<220>
   <221> Primer
   <222> (1)..(28)
   <223> aldA1
<400> 1
   ccgaaaacaa acatctagat cacaggag 28
<210> 2
   <211> 24
   <212> DNA
   <213> synthetic sequence
<220>
   <221> Primer
   <222> (1)..(24)
   <223> aldA2
<400> 2
   cctccgaagc tttcactcat taag 24
<210> 3
   <211> 25
   <212> DNA
   <213> synthetic sequence
<220>
   <221> Primer
   <222> (1)..(25)
   <223> aldB1
<400> 3
   caactatctc tagaaccctt gcccg 25
<210> 4
   <211> 24
   <212> DNA
   <213> synthetic sequence
<220>
   <221> Primer
   <222> (1)..(24)
   <223> aldB2
<400> 4
   ccaatgcgac aagcttctta tatc 24
<210> 5
   <211> 29
   <212> DNA
   <213> synthetic sequence
<220>
   <221> Primer
   <222> (1)..(29)
   <223> aldH3
<400> 5
   agccgtcgac accacgcaaa cgtcgcagg 29
<210> 6
   <211> 29
   <212> DNA
   <213> synthetic sequence
<220>
   <221> Primer
   <222> (1)..(29)
   <223> aldH5
<400> 6
   atcggatcct ctgcgccctg acgttcaca 29
<210> 7
   <211> 26
   <212> DNA
   <213> synthetic sequence
<220>
   <221> Primer
   <222> (1)..(26)
   <223> betB1
<400> 7
   cagcatctag acaccgatta accgag 26
<210> 8
   <211> 27
   <212> DNA
   <213> synthetic sequence
<220>
   <221> Primer
   <222> (1)..(27)
   <223> betB2
<400> 8
   caaattgcaa ataaagcttc tggttag 27